# EUROPEAN PATENT APPLICATION

(11) **EP 4 821 941 A1**
(43) Date of publication of application: **14.10.2026**
(21) Application number: 24900642.0
(22) Date of filing: 04.12.2024
(51) Int. Cl.: C07K 7/06, A61K 45/00, A61K 47/59, A61K 47/62, A61K 49/00, A61P 25/00, C12Q 1/02

(54) **BRAIN-TARGETING PEPTIDE, TRANSPORT CARRIER, AND METHOD FOR SCREENING PEPTIDE BINDING TO TARGET CELL**

(30) Priority: 04.12.2023 JP 2023204750
(71) Applicant: Mitsui Chemicals, Inc., Tokyo 104-0028 (JP)
(72) Inventor: KOJIMA Hideto, Otsu-shi, Shiga 520-2192 (JP); KATAGI Miwako, Otsu-shi, Shiga 520-2192 (JP)
(74) Representative: Mewburn Ellis LLP
(86) International application number: PCT/JP2024/042865
(87) International publication number: WO 2025/121348

(57) **Abstract**

The present invention provides a cerebrum-targeting peptide including an amino acid sequence represented by SEQ ID NO: 1, and a method for screening a peptide that binds to a target cell in a non-human organism, comprising contacting a peptide library consisting of 7-amino acid peptides with a target cell to recover a group of peptides that bind to the target cell to obtain a peptide population that binds to the target cell, and selecting, from the peptide population, a peptide consisting of an amino acid sequence that is identical to a portion of a protein expressed in the non-human organism and that is also identical to a portion of a protein constituting a virus, as a peptide that binds to the target cell.

## Description

### TECHNICAL FIELD

The present invention relates to a peptide targeting a cerebrum, a transport carrier, and a pharmaceutical composition utilizing the peptide. The present invention also relates to a method for screening a peptide that binds to a target cell.

This application claims priority based on Japanese Patent Application No. 2023-204750, filed on December 4, 2023, the contents of which are incorporated herein by reference.

### BACKGROUND ART

Techniques for targeting specific organs are expected to be applied to, for example, drug delivery systems (DDS) for delivering medicines. For example, transport efficiency can be improved by modifying a transport carrier targeted to a specific tissue with a peptide that specifically binds to cells constituting the tissue. Examples of the peptides targeting specific tissues or cells include a neovascularization-specific peptide described in Patent Document 1, a cardiac-specific peptide described in Patent Document 2, and a spinal cord tissue-specific peptide described in Patent Document 3.
Furthermore, Patent Document 4 describes a peptide that can penetrate a blood-brain barrier and that is used to modify a transport carrier targeting a brain.

### Citation List

### Patent Documents

Patent Document 1: PCT International Publication No. WO2000/023476
Patent Document 2: U.S. Patent No. 6,303,573
Patent Document 3: Japanese Patent No. 6479331
Patent Document 4: Japanese Patent No. 7378046

### SUMMARY OF INVENTION

### Technical Problem

An objective of the present invention is to provide a peptide that specifically binds to cells that constitute a cerebrum and that is suitable for modifying transport carriers targeting the cerebrum, a transport carrier targeting the cerebrum including the peptide, and a method for screening a peptide that binds to a target cell.

### Solution to Problem

The present invention includes the following aspects.
[1] A cerebrum-targeting peptide comprising an amino acid sequence represented by SEQ ID NO: 1 (EVGTARY).
[2] A transport carrier for specifically transporting to a cerebrum, wherein the transport carrier is a complex in which a cerebrum-targeting peptide comprising an amino acid sequence represented by SEQ ID NO: 1 and a cationic polymer are linked.
[3] The transport carrier according to [2], wherein the cerebrum is a cerebral nerve cell.
[4] A pharmaceutical composition, comprising the cerebrum-targeting peptide according to [1], the transport carrier according to [2], or the transport carrier according to [3].
[5] The pharmaceutical composition according to [4], further comprising a therapeutic agent or a detection agent.
[6] A method for screening a peptide that binds to a target cell in a non-human organism, comprising:
   contacting a peptide library consisting of 7-amino acid peptides with a target cell to recover a group of peptides that bind to the target cell to obtain a peptide population that binds to the target cell; and
   selecting, from the peptide population, a peptide consisting of an amino acid sequence that is identical to a portion of a protein expressed in the non-human organism and that is also identical to a portion of a protein constituting a virus, as a peptide that binds to the target cell.
[7] The method for screening a peptide that binds to a target cell according to [6], wherein
   a group of peptides that specifically bind to the target cells are recovered from the peptide population, and a peptide consisting of an amino acid sequence that is identical to a portion of a protein expressed in the non-human organism and that is also identical to a portion of a ligand recognized by a virus during infection is selected from the group of peptides as the peptide that binds to the target cell.

### Advantageous Effects of Invention

According to the present invention, it is possible to provide a peptide targeting a cerebrum, and a transport carrier including the peptide targeting the cerebrum.

Furthermore, according to the present invention, it is possible to efficiently screen for peptides that specifically bind to specific tissues or cells in a living body and that are suitable for modifying transport carriers used in DDS.

### BRIEF DESCRIPTION OF DRAWINGS

FIG. 1A is a nuclear staining image and a dsRed fluorescence image of a cerebrum of a mouse administered with a nucleic acid-PEI-P_{CN} complex in Example 2.
FIG. 1B is a nuclear staining image and a dsRed fluorescence image of a cerebrum of a mouse administered with a nucleic acid-PEI-Benzyl complex in Example 2.
FIG. 1C is a nuclear staining image and a dsRed fluorescence image of a cerebrum of a mouse administered with dsRed in Example 2.
FIG. 2A is a nuclear staining image and a dsRed fluorescence image of a heart of a mouse administered with a nucleic acid-PEI-P_{CN} complex in Example 2.
FIG. 2B is a nuclear staining image and a dsRed fluorescence image of a heart of a mouse administered with a nucleic acid-PEI-Benzyl complex in Example 2.
FIG. 2C is a nuclear staining image and a dsRed fluorescence image of a heart of a mouse administered with dsRed in Example 2.
FIG. 3A is a nuclear staining image and a dsRed fluorescence image of a small intestine of a mouse administered with a nucleic acid-PEI-P_{CN} complex in Example 2.
FIG. 3B is a nuclear staining image and a dsRed fluorescence image of a small intestine of a mouse administered with a nucleic acid-PEI-Benzyl complex in Example 2.
FIG. 3C is a nuclear staining image and a dsRed fluorescence image of a small intestine of a mouse administered with dsRed in Example 2.
FIG. 4A is a nuclear staining image and a dsRed fluorescence image of a liver of a mouse administered with a nucleic acid-PEI-P_{CN} complex in Example 2.
FIG. 4B is a nuclear staining image and a dsRed fluorescence image of a liver of a mouse administered with a nucleic acid-PEI-Benzyl complex in Example 2.
FIG. 4C is a nuclear staining image and a dsRed fluorescence image of a liver of a mouse administered with dsRed in Example 2.

### DETAILED DESCRIPTION OF THE INVENTION

Hereinafter, embodiments of the present invention will be specifically described.

In the present invention and the present specification, "X1 to X2 (X1 and X2 are real numbers satisfying X1<X2)" means "greater than or equal to X1 and less than or equal to X2".

In the present invention and the present specification, the term "polypeptide" refers to a polymer of 25 or more amino acids bonded together by peptide bonds, and the term "peptide" refers to a polymer of 2 to 24 amino acids bonded together by peptide bonds.

In the present invention and the present specification, the term "comprise" means that it may contain components other than the target component. The term "consist of" means that it does not contain components other than the target component. The term "consist essentially of" means that it does not contain components other than the target component in a form that exerts a special function (such as a form that completely loses the effects of the invention). In the present invention and the present specification, when "comprise" is written, it includes both an embodiment that "consists of" and an embodiment that "consists essentially of".

In the present invention and the present specification, "targeting (specific cells or tissues)" means targeting specific cells or tissues as the binding target. For example, a "(specific cell or tissue) targeting peptide" means a peptide that binds to a specific cell or tissue. A "(specific cell or tissue) targeted transport carrier" means a transport carrier for transporting a substance to a specific cell or tissue.

In the present invention and the present specification, the term "specifically bind (to a specific cell or tissue)" means that the affinity (strength of binding) for a specific cell or tissue is higher than the affinity for the majority of other cells or tissues.

### <Cerebrum-targeting peptide>

The cerebrum-targeting peptide of the present embodiment is a peptide comprising an amino acid sequence represented by SEQ ID NO: 1 (EVGTARY). A peptide consisting of the amino acid sequence represented by SEQ ID NO: 1 is a peptide that specifically binds to cells constituting cerebral tissue, particularly cerebral nerve cells. Examples of the cerebral nerve cells include cortical neurons, striatal neurons, hippocampal neurons, hypothalamic neurons, PVN neurons, and substantia nigra neurons. By comprising the amino acid sequence represented by SEQ ID NO: 1, the cerebrum-targeting peptide of the present embodiment can specifically bind to cells constituting cerebral tissue and function as a peptide targeting the cerebrum.

The cerebrum-targeting peptide of the present embodiment may be a peptide consisting solely of the amino acid sequence represented by SEQ ID NO: 1, or may contain other amino acid sequence portions. The other amino acid sequence portions are not particularly limited, as long as they do not impair the specific binding ability of the amino acid sequence portion represented by SEQ ID NO: 1 to the cerebrum. Examples of the other amino acid sequence portions include various signal peptides and tag peptides. These signal peptides and tag peptides can be appropriately selected from various signal peptides and tag peptides commonly used in the production of recombinant proteins. Examples of the tag peptides include His tags, HA (hemagglutinin) tags, Myc tags, and Flag tags. The other amino acid sequence portions preferably include linkers for binding to other substances or amino acid residues that contribute to binding to other substances. Examples of the amino acid residues that contribute to binding to other substances include cysteine residues.

The cerebrum-targeting peptide of the present embodiment may be a linear or cyclic peptide. Cyclization of the peptide is expected to improve hydrolysis resistance and in vivo stability.

The cerebrum-targeting peptide of the present embodiment may be modified in various ways as long as the specific binding ability to the cerebrum due to the amino acid sequence portion represented by SEQ ID NO: 1 is not impaired. Examples of such modifications include acetylation of the N-terminus and amidation of the C-terminus.

The cerebrum-targeting peptide of the present embodiment may be a peptide consisting only of L-amino acids, a peptide containing both L-amino acids and D-amino acids, or a peptide consisting only of D-amino acids. Furthermore, some or all of the amino acids constituting the cerebrum-targeting peptide of the present embodiment may be replaced with artificial amino acids.

The cerebrum-targeting peptide of the present embodiment can be easily synthesized by commonly used peptide synthesis techniques, and can also be easily produced by using commonly used expression systems such as Escherichia coli.

The cerebrum-targeting peptide of the present embodiment may be bound to a substance other than a peptide or polypeptide. The substance can be appropriately selected from various labeling substances used to label peptides. Examples of the labeling substance include low-molecular-weight compounds such as biotin, fluorescent substances, etc.

When the cerebrum-targeting peptide of the present embodiment is a substance in which a peptide or polypeptide containing the amino acid sequence represented by SEQ ID NO: 1 is linked to a labeling substance, the two substances may be linked directly or indirectly via a linker. The linker is not particularly limited as long as it is a divalent or higher linking group that does not impair the specific binding ability of the amino acid sequence portion represented by SEQ ID NO: 1 to the cerebrum. Examples of the linking group include a hydrocarbon group, -NH-, -O-, -CO-, -S-, -SO-, a divalent or higher aromatic ring group, a maleimide group, and combinations of two or more of these. The peptide or polypeptide containing the amino acid sequence represented by SEQ ID NO: 1 can be linked to a labeling substance using chemical reactions commonly used in the synthesis of chemical substances.

The cerebrum-targeting peptide of the present embodiment can be bound to the cerebrum or cells constituting the cerebrum by contacting the cerebrum-targeting peptide of the present embodiment with the cerebrum or cells constituting the cerebrum. Contact of the cerebrum-targeting peptide of the present embodiment with the cerebrum or cells constituting the cerebrum may be performed in vivo, in vitro, or ex vivo. The method for contacting the cerebrum-targeting peptide of the present embodiment with the cerebrum or cells constituting the cerebrum is not particularly limited. For example, when contacting the cerebrum-targeting peptide of the present embodiment in vitro or ex vivo, an appropriate amount of the peptide may be contacted with the cerebrum or a subject containing the cerebrum, and then incubated as necessary. Furthermore, when targeting the cerebrum-targeting peptide of the present embodiment in vivo, it can be performed by direct injection into the cerebrum, intravenous, subcutaneous, intramuscular, or peritoneal injection, as well as oral administration, inhalation administration, transmucosal administration, etc.

The cerebrum-targeting peptide of the present embodiment specifically binds to the cerebrum and the cells that make up the cerebrum, and is therefore useful for the observation and diagnosis of cerebral tissue. The peptide can be used for various applications in the pharmaceutical field, such as molecular imaging of cerebral tissue, prevention, alleviation, or treatment of diseases in cerebral tissue, and as a tool for evaluating the therapeutic effects of diseases. Diseases in cerebral tissue include, for example, cerebral neurological diseases such as Alzheimer's disease and Parkinson's disease, and brain tumors. Furthermore, when the cerebrum-targeting peptide of the present embodiment is a peptide linked to a labeling substance such as a fluorescent substance, the cerebrum-targeting peptide can be used to label the cerebrum and the cells that make up the cerebrum, and is useful for the observation and diagnosis of the cerebrum.

The biological species of the cerebrum and cells constituting the cerebrum targeted by the cerebrum-targeting peptide of the present embodiment are not particularly limited. Examples of the animals include vertebrates such as mammals, birds, reptiles, amphibians, and fish. Examples of the mammals include rodents such as mice, rats, hamsters, and guinea pigs; ungulates such as pigs, cows, goats, horses, and sheep; carnivores such as dogs and cats; and primates such as rhesus monkeys, cynomolgus monkeys, marmosets, orangutans, chimpanzees, and humans.

### <Transport carriers targeting cerebrum>

The transport carrier of the present embodiment is a transport carrier (cerebrum-targeted transport carrier) for transporting specifically to the cerebrum, and is a complex formed by linking a cerebrum-targeting peptide containing the amino acid sequence represented by SEQ ID NO: 1 with a cationic polymer. The transport carrier of the present embodiment utilizes the specific binding ability to the cerebrum due to the amino acid sequence portion represented by SEQ ID NO: 1, and is therefore suitable for use in delivering a substance to be delivered to cerebral tissue. The transport carrier of the present embodiment can transport a substance to cerebral tissue more efficiently, thereby allowing the beneficial effects of the substance to be delivered to be efficiently exerted in spinal cord tissue. Furthermore, the transport carrier of the present embodiment can reduce or avoid side effects on tissues and cells other than cerebral tissue.

The cerebrum-targeting peptide in the transport carrier of the present embodiment can be the cerebrum-targeting peptide of the present embodiment. Furthermore, the transport carrier of the present embodiment may be a complex in which the cerebrum-targeting peptide and the cationic polymer are directly linked, or in which the cerebrum-targeting peptide and the cationic polymer are indirectly linked via a linker. The linker is not particularly limited, as long as it is a divalent or higher linking group that does not impair the specific binding ability of the amino acid sequence portion represented by SEQ ID NO: 1 to the cerebrum. Examples of such linking groups include hydrocarbon groups, -NH-, -O-, -CO-, -S-, -SO-, divalent or higher aromatic ring groups, maleimide groups, and combinations of two or more of these. Linking the cerebrum-targeting peptide to the cationic polymer, or linking the cerebrum-targeting peptide, the cationic polymer, and the linker can be performed using chemical reactions commonly used in the synthesis of chemical substances.

Cationic polymers in the transport carrier of the present embodiment are relatively easy to chemically modify and are widely used, and therefore include DEAE-dextran, Dendrimer, Polybrene (registered trademark) (CAS: 28728-55-4), Polyethyleneimine (PEI), and various derivatives thereof. Instead of the cationic polymer, a complex in which calcium phosphate or a cationic lipid is linked to a cerebrum-targeting peptide can also be used as a cerebral targeting transport carrier.

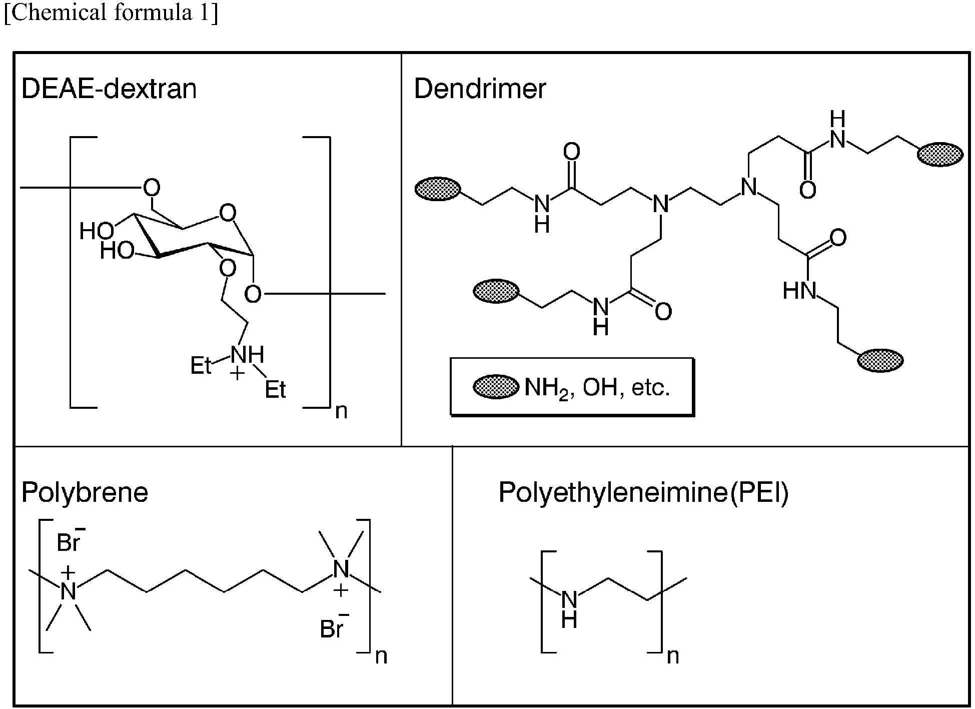

The transport carrier of the present embodiment may be directly or indirectly linked to the substance to be transported, or may not be linked. When the surface of the substance to be transported is anionic, the transport carrier of the present embodiment and the substance to be transported can be mixed in an appropriate solvent to form a complex through electrostatic interaction with the cationic polymer moiety in the transport carrier. The complex formed between the transport carrier and the substance to be transported can be used to specifically transport the substance to the cerebrum.

Examples of the substances that can be transported by the transport carrier of the present embodiment include nucleic acids, peptides, proteins (e.g., protein-based medicines such as antibodies, antibody fragments, antagonists, and agonists), lipids, peptide lipids, sugars, low-molecular-weight compounds, and other synthetic or natural compounds. These substances can be used alone or in combination of two or more. The size of the substance to be transported is not particularly limited, as long as it is large enough to be introduced into cells that make up the cerebrum using the transport carrier of the present embodiment.

Among the substances to be transported by the transport carrier of the present embodiment, nucleic acids are particularly preferable. Examples of the nucleic acids include DNA such as plasmid DNA, cDNA, and antisense DNA, and RNA such as siRNA, shRNA, miRNA, antisense RNA, mRNA, tRNA, and rRNA. Examples of such nucleic acids include nucleic acids that serve as active ingredients in gene therapy and mRNA vaccine therapy.

### <Pharmaceutical composition>

The pharmaceutical composition of the present embodiment includes the cerebrum-targeting peptide of the present embodiment or the transport carrier of the present embodiment. For example, a transport carrier of the present embodiment containing a therapeutic agent or a detection agent targeted to the cerebrum as a transported substance is suitable as an active ingredient of a pharmaceutical composition targeting the cerebrum. For example, by using a medicine that acts as a therapeutic agent among substances targeted to the cerebrum as a transported substance and the transport carrier of the present embodiment, the therapeutic agent can be efficiently transported to the cerebrum, and improved therapeutic effects can be expected. Furthermore, by using a detection agent for detecting the cerebrum as a transported substance and the transport carrier of the present embodiment, the cerebrum can be efficiently detected. Similarly, among the cerebrum-targeting peptides of the present embodiment, peptides in which a therapeutic agent or a detection agent targeted to the cerebrum as a target tissue is linked directly or indirectly to a peptide moiety consisting of the amino acid sequence represented by SEQ ID NO: 1 are also suitable as active ingredients of pharmaceutical compositions targeting the cerebrum.

The content of the substance to be delivered in the pharmaceutical composition of the present embodiment is not limited as long as the useful effect of the substance to be delivered is exerted in cerebral tissue, and can be appropriately determined by a person skilled in the art depending on the form of the pharmaceutical composition, the manner of use, the weight, type, and condition of the cerebral tissue, etc.

The pharmaceutical composition of the present embodiment may consist solely of the cerebrum-targeting peptide of the present embodiment or the transport carrier of the present embodiment, or may contain a pharmaceutically acceptable carrier, if necessary. The carrier is not limited as long as it achieves the effects of the present invention, and examples thereof include purified water, buffer solution, physiological saline, RNase-free water, DNase-free water, protease-free water, aqueous glucose solution, isotonicity agent, excipient, binder, lubricant, disintegrant, fluidizing agent, diluent, thickener, stabilizer, buffer, preservative, antioxidant, flavoring agent, colorant, etc., and the carrier can be appropriately selected by those skilled in the art depending on the form of use.

The dosage form of the pharmaceutical composition of the present embodiment is not particularly limited and can be appropriately selected from commonly used dosage forms, taking into consideration the administration method, etc. Examples of such dosage forms include tablets (including plain tablets, sugar-coated tablets, effervescent tablets, film-coated tablets, chewable tablets, troches, etc.), capsules, pills, powders (pulverized medicines), granules, fine granules, liquids, suspensions, emulsions, pastes, syrups, and injections (including those prepared as liquids by mixing with distilled water or infusions such as amino acid infusions or electrolyte infusions at the time of use).

The method of administration of the pharmaceutical composition of the present embodiment is not particularly limited, and can be, for example, intra-arterial administration, intravenous administration, intraoral administration, rectal administration, enteral administration, transdermal administration, oral administration, etc.

The pharmaceutical composition of the present embodiment is to be administered to vertebrates having a cerebrum, preferably to mammals, and particularly preferably to primates including humans.

The amount of the cerebrum-targeting peptide of the present embodiment or the transport carrier of the present embodiment contained in the pharmaceutical composition of the present embodiment is not particularly limited. The pharmaceutical composition of the present embodiment is preferably adjusted so that a sufficient amount of the target substance can be delivered to the cerebrum by the cerebrum-targeting peptide and the transport carrier. The dosage of the pharmaceutical composition of the present embodiment can be appropriately determined depending on various conditions such as a patient's weight, age, sex, symptoms, administration route, and dosage form.

### <Method for screening peptides that bind to target cells>

The screening method of the present embodiment is a method for screening peptides that bind to target cells in a non-human organism. Specifically, first, (A) a peptide library consisting of 7-amino acid peptides is contacted with the target cells, and a group of peptides that bind to the target cells is recovered, and the recovered group of peptides is designated as a peptide population that binds to the target cells. Next, (B) from the peptide population, peptides that have the same amino acid sequence as a portion of a protein expressed in the non-human organism and the same amino acid sequence as a portion of a protein that constitutes a virus are selected as peptides that bind to the target cells.

In (A), the method for contacting target cells with a peptide library consisting of 7-amino acid peptides is not particularly limited. For example, a phage population displaying the peptide library may be administered to a non-human organism, followed by recovery of tissue containing target cells from the non-human organism, and, if necessary, isolation of the target tissue from the recovered tissue, followed by recovery of peptides derived from the administered peptide library. Alternatively, a phage population displaying the peptide library may be introduced into a culture medium for target cells and incubated, followed by recovery of peptides bound to the target cells. Recovery of tissues or cells, and recovery of peptides from the recovered tissue, etc., can be performed by conventional methods.

The phage used is not particularly limited as long as it is one that is commonly used in phage display libraries, and the examples thereof include the M13 phage. As the phage population that presents the peptide library, a phage display library can be used in which the gene encoding the peptide library is inserted into the gene encoding the pIII coat protein of the M13 phage. As the peptide library, a library of random peptides consisting of 7 amino acids is preferable.

Non-human living organisms are not particularly limited, and include animals, plants, etc. Examples of the animals include vertebrates and invertebrates. Examples of the vertebrates include mammals, birds, reptiles, amphibians, fish, etc. Examples of the mammals include rodents such as mice, rats, hamsters, and guinea pigs; ungulates such as pigs, cows, goats, horses, and sheep; carnivores such as dogs and cats; and primates such as rhesus monkeys, cynomolgus monkeys, marmosets, orangutans, chimpanzees, and humans. Examples of the invertebrates include arthropods and mollusks.

The phage population can be administered to a non-human organism using a method appropriate for the non-human organism and the target cells. The target cells may be a cell population of all cells constituting an organ or tissue, or a single cell. For example, a phage population displaying a peptide library is administered to a non-human organism, and then the cerebrum of the non-human organism is collected. Peptides derived from the administered peptide library are then recovered from the cerebrum, thereby obtaining a peptide population that binds to cerebral cells (including all the various types of cells constituting the cerebrum). Specific cells, such as cortical neurons, striatal neurons, hippocampal neurons, hypothalamic neurons, PVN neurons, and substantia nigra neurons, are isolated from the collected cerebrum, and peptides derived from the peptide library are recovered from each isolated cell, thereby obtaining a peptide population that binds to a specific single cell.

The collected peptide group can be identified by genetic analysis of the phage displaying the peptide. Specifically, the base sequences encoding the peptides displayed by the collected phage population are decoded using a next-generation sequencer to obtain a population of reads. The peptides encoded by the obtained population of reads are peptides that specifically bind to a target site in a body.

A peptide group prepared in (A) is again contacted with target cells, and the peptide population binding to the target cells is recovered. By repeating this process, a peptide population with higher affinity for target cells can be obtained (panning). By repeating this process a sufficient number of times, for example, two or more times, preferably three or more times, more preferably four or more times, a peptide group consisting of peptides that specifically bind to target cells can be obtained.

Peptides that have binding affinity to target cells may also have binding affinity to sites other than the target cells. By using a next-generation sequencer, peptides that have binding affinity to cells other than the target cells can be removed from the data (in silico) (virtual subtraction). By performing virtual subtraction, the sensitivity and specificity of the binding affinity of the resulting peptide group to the target cells can be significantly improved, resulting in a peptide group consisting of peptides that specifically bind to the target cells.

From the peptide population prepared in (A), peptides that have the same amino acid sequence as a portion of a protein expressed in the non-human organism and the same amino acid sequence as a portion of a protein constituting a virus are selected as peptides that bind to the target cells (B). The peptides in the peptide population prepared in (A) consist of seven amino acids. A "peptide with the same amino acid sequence as a portion of a protein" refers to a peptide that consists of an amino acid sequence that is completely identical to the amino acid sequence of a portion (7 amino acids) of the full-length amino acid sequence of the protein. The full-length of the protein is not particularly limited as long as it is more than seven amino acids, and may be a peptide of about 10 amino acids or a protein consisting of several thousand amino acids.

Peptides with an amino acid sequence identical to a portion of a protein expressed in a non-human organism have low immunogenicity to the non-human organism and can be administered relatively safely. Furthermore, peptides with an amino acid sequence identical to a portion of a protein constituting a virus are expected to have an effect similar to that of the virus when administered in vivo. For example, a peptide with an amino acid sequence identical to a portion of a protein constituting a virus known to infect the same species of organism as the target cells is expected to infect the target cells as easily as the virus. In the screening method of the present embodiment, it is particularly preferable to select, from the peptide population prepared in (A), a peptide with an amino acid sequence identical to a portion of a protein expressed in a non-human organism and having an amino acid sequence identical to a peptide or a portion thereof known as an infectious ligand for the virus, as a peptide that binds to target cells.

While preferable embodiments of the present invention have been described above, it should be understood that these are illustrative of the present invention and should not be construed as limiting. Additions, omissions, substitutions, and other modifications can be made without departing from the spirit or scope of the present invention. Accordingly, the present invention is not to be deemed limited by the foregoing description, but is limited only by the scope of the appended claims.

### (Examples)

The present invention will now be described in more detail with reference to examples, but the present invention is not limited to the following examples.

### [Experimental Example 1]

A peptide that specifically binds to cortical neurons in a mouse brain was identified.

### (In vivo panning using mice)

Mice were administered 1×10¹¹ cfu of the phage library via a tail vein. A commercially available kit (product name "Ph.D^{™}-7 Phage Display Peptide Library Kit", New England Biolabs) was used as the phage library. This phage library expressed random peptides consisting of seven amino acids on the phage surface.

Five minutes after administration of the phage library, mice were anesthetized, cardiac blood was collected, and the mice were then perfused and bled with phosphate-buffered saline (PBS). Subsequently, target tissues were collected from the mice. Thirty-seven organs and 152 types of cells were collected from the target tissues. Cells were collected by laser capture microdissection (LCM).

Next, each of the collected target sites was homogenized to obtain a phage solution. The phage solution was infected into Escherichia coli, added to top agar, and plated on an LB plate. The cells were cultured overnight at 37°C. After confirming the phage plaques, the phages were recovered and propagated using SM buffer (50 mM Tris-HCl (pH 7.5), 100 mM NaCl, 8 mM MgSO_{4,} 0.01% gelatin). PCR amplification was performed using the SM buffer as a sample, and the PCR products were electrophoresed, gel extracted, and purified, and samples for next-generation sequencing were prepared for each target site.

Subsequently, the phages collected for each target site were mixed and administered again to mice via the tail vein at 1×10¹¹ cfu, and a second in vivo panning was performed in the same manner as above to prepare samples for next-generation sequencing for each target site.

The phages recovered from each target site in the second in vivo panning were mixed and administered again to mice via the tail vein at 1×10¹¹ cfu. A third in vivo panning was performed in the same manner as above to prepare samples for next-generation sequencing.

Next, the base sequences of the prepared samples for next-generation sequencing were analyzed using a next-generation sequencer.

### (Analysis results by next-generation sequencing)

Focusing on cerebral cortical neurons, the amino acid sequences of peptides with specific binding to cerebral cortical neurons were analyzed based on the results of next-generation sequencing after the third round of panning.

Of the 152 cells, a group of peptides that bound only to cerebral cortical neurons was selected. From this group of peptides, five peptides with the highest number of detected reads were selected based on the amino acid sequences of the peptides expressed on the surface of phages adsorbed to cerebral cortical neurons. The five selected peptides (P1) to (P5) are shown in Table 1. These five peptides (P1) to (P5) were subjected to a NCBI protein BLAST search to identify proteins containing each amino acid sequence. As a result, only peptide (P3) had an amino acid sequence identical to a portion of a mouse protein (TGF-β type 2 receptor) and a portion of a protein constituting a virus (serine/threonine kinase). These results suggested that peptide P3 is highly expressed throughout the cerebrum, and administration to mice did not produce antibodies, suggesting that peptide P3 is easily taken up into cells. Therefore, a peptide (CEVGTARYC: SEQ ID NO: 6) in which cysteine residues were added to both termini of peptide P3 was used as a cerebrum-targeting peptide P_{CN} in subsequent experiments.

**[Table 1]**

| Peptide | Amino acid sequence | SEQ ID NO | Number of reads | | Mouse protein | Viral protein |
|---|---|---|---|---|---|---|
| | | | Whole cerebrum | Cortical neurons | | |
| P1 | HLDSTPH | 2 | 140 | 247 | None | None |
| P2 | KVTTSGI | 3 | 23 | 216 | None | None |
| P3 | EVGTARY | 1 | 41 | 135 | TGF-β type 2 receptor | serine/threonine kinase |
| P4 | TGOGGSK | 4 | 71 | 118 | None | None |
| P5 | TDRAPHR | 5 | 19 | 104 | None | None |

In this experiment, none of the blood-brain barrier-permeable peptides described in Patent Document 4 were obtained. The blood-brain barrier-permeable peptides described in Patent Document 4 were obtained by screening peptides that bind to hCMEC/D3 cells in vitro, and it is presumed that this is because, when administered in vivo, they were adsorbed to blood vessels and did not reach the brain.

### [Experimental Example 2]

A cationic polymer was linked via a linker to the cerebrum-targeting peptide P_{CN} (SEQ ID NO: 6) obtained in Example 1 to prepare a transport carrier for specifically transporting the peptide to the cerebrum.

### (Preparation of cationic polymer)

Branched Polyethyleneimine (BPEI_{25,000}) (manufactured by Sigma-Aldrich) with a molecular weight of 25,000 was used as the cationic polymer.

Specifically, as shown in the following chemical reaction formula, the -OH group of 5-hexynoic acid was chlorinated with thionyl chloride, and then reacted with BPEI_{25,000} to synthesize BPEI_{25,000}-Alkyne. The alkyne introduction rate of the obtained BPEI_{25,000}-Alkyne was 4%.

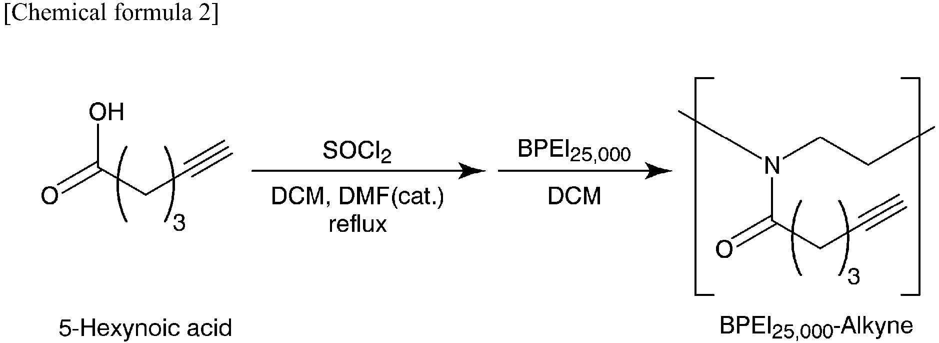

### (Formation of a complex between a cerebrum-targeting peptide and a cationic polymer)

The cysteine residue at the C-terminus of P_{CN} was linked to a solid support, and the reactive groups of the side chains, i.e., -SH of the cysteine residue, -OH of the tyrosine residue, -OH of the threonine residue, -NH₂ of the arginine residue, and -COOH of the glutamic acid residue, were protected. Next, an azide linker, in which the azide group and carboxy group were linked via a polyethylene glycol chain, was linked to the amino group at the N-terminus of the protected P_{CN}. Subsequently, all of the protecting groups in the side chains of P_{CN} were deprotected to obtain azide-modified P_{CN}.

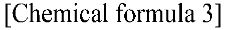

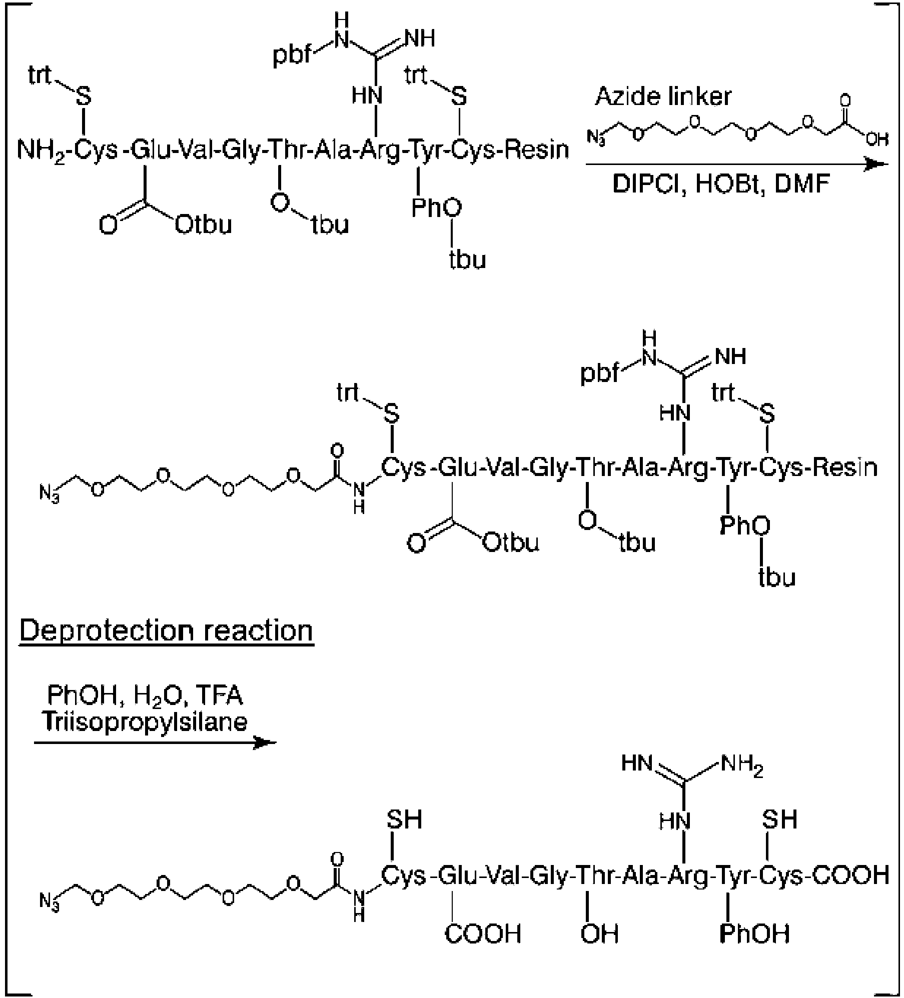

Next, the azide group of the azide-modified P_{CN} was linked to the alkynyl group of BPEI_{25,000}-Alkyne by click reaction to synthesize PEI-P_{CN}.

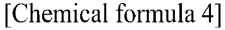

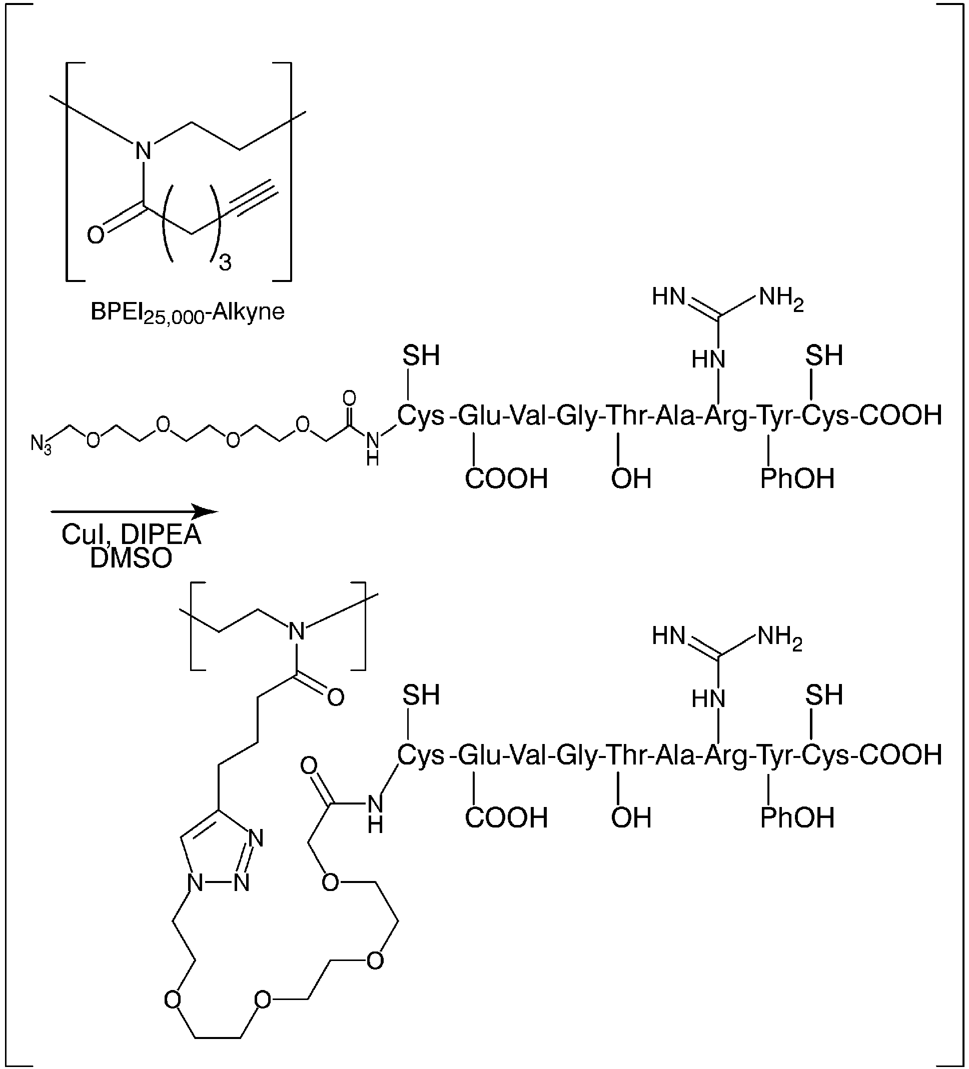

### (Formation of linked cationic polymer)

PEI-Benzyl was synthesized by linking the alkynyl group of BPEI_{25,000}-Alkyne to the azide group of an azide linker, which consists of an azide group and N-benzylacrylamide linked by a polyethylene glycol chain, via a click reaction.

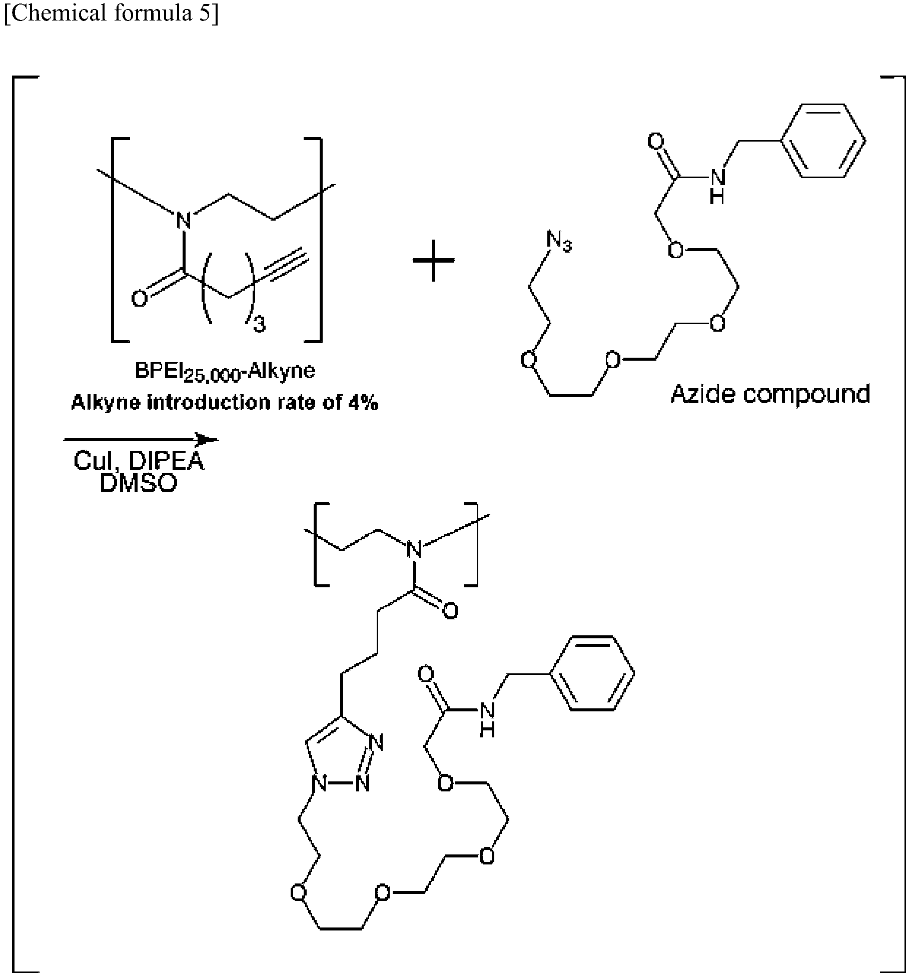

### (Uptake of nucleic acid and PEI-P_{CN} complex into mouse brain)

Using PEI-P_{CN} as a transport carrier, a nucleic acid was administered to mice, and the uptake into the cerebrum and other tissues was examined. The nucleic acid used was the fluorescent protein vector dsRed plasmid (6155 bp, a modified version of a plasmid donated by Dr. Oka Kazuhiro, Department of Molecular and Cellular Biology, Baylor College of Medicine, Houston, Texas, USA; the same type is available commercially from Takara).

When 200 µg of dsRed plasmid was mixed with PEI-P_{CN} at various concentrations in phosphate-buffered saline (PBS), it was confirmed that the dsRed plasmid and PEI-P_{CN} formed a complex (nucleic acid-PEI-P_{CN} complex) at a molar ratio of dsRed plasmid:PEI-P_{CN} = 1:200. Furthermore, when 200 µg of dsRed plasmid was mixed with PEI-Benzyl at various concentrations, it was confirmed that the dsRed plasmid and PEI-Benzyl formed a complex (nucleic acid-PEI-Benzyl complex) at a molar ratio of dsRed:PEI-Benzyl = 1:50.

Mice were intravenously injected with each of the complexes, resulting in a dsRed plasmid dose of 3×10¹¹ particles per mouse, and then housed for four days. For comparison, mice were also administered an equivalent amount of dsRed plasmid alone. No health problems were observed in any of the mice, and their food intake remained normal, as before administration. Four days after administration, brains, hearts, small intestines, and livers were collected from the mice, and formalin-fixed, paraffin-embedded sections were prepared according to standard procedures. Nuclei in each tissue section were stained with DAPI (4',6-diamidino-2-phenylindole), and blue fluorescent images (nuclear staining images) and red fluorescent images (dsRed fluorescent images) were captured to examine the expression status of dsRed in each tissue.

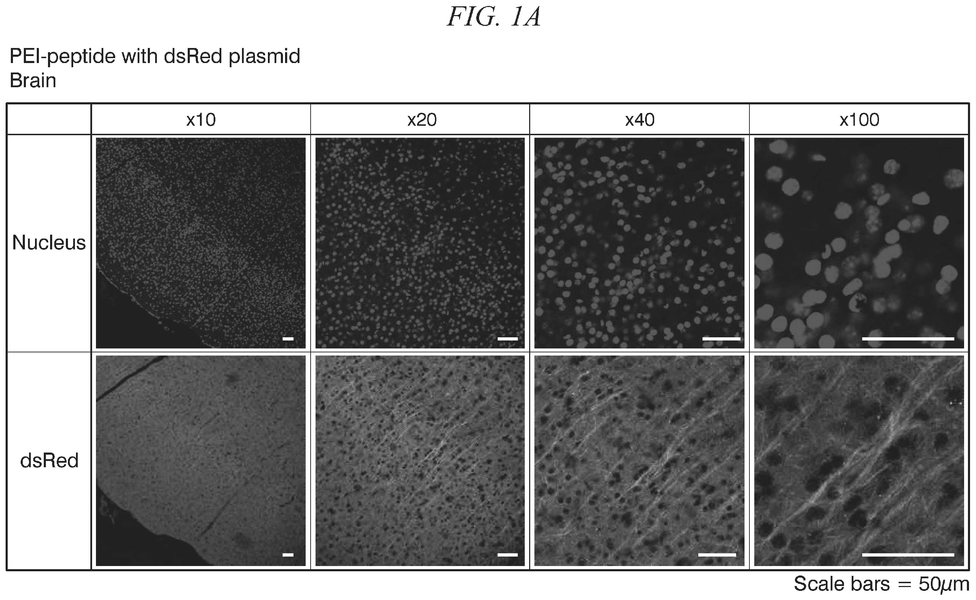

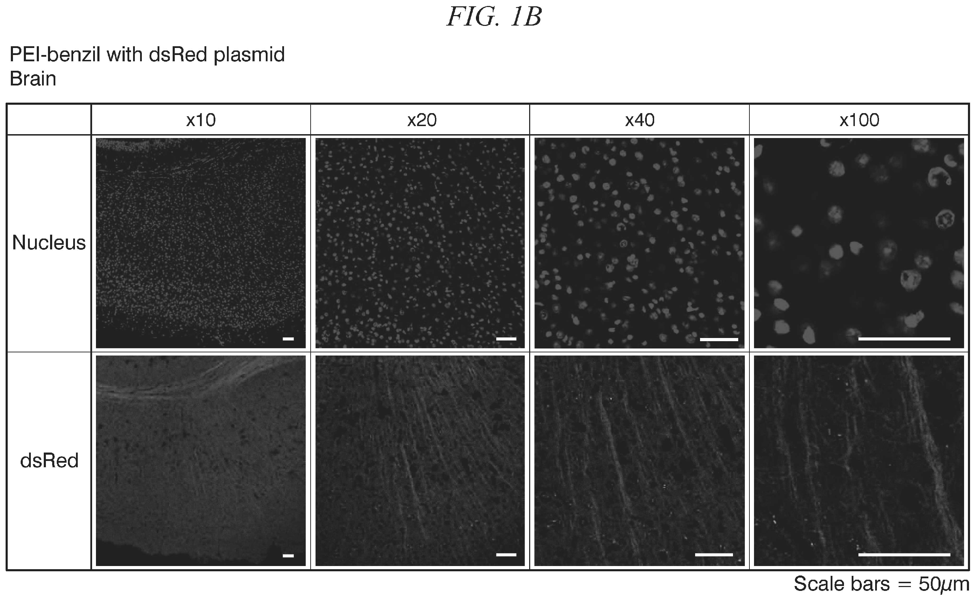

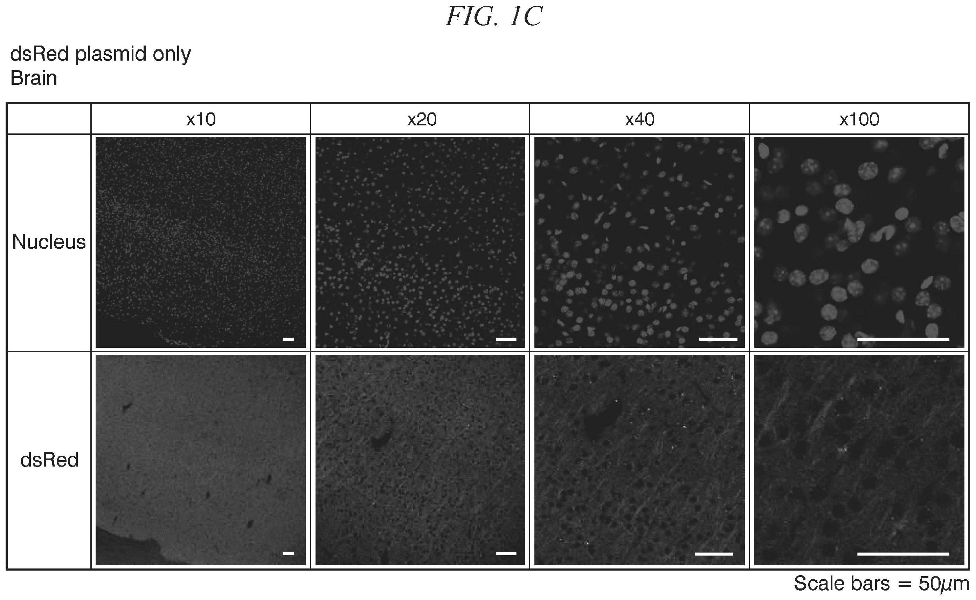

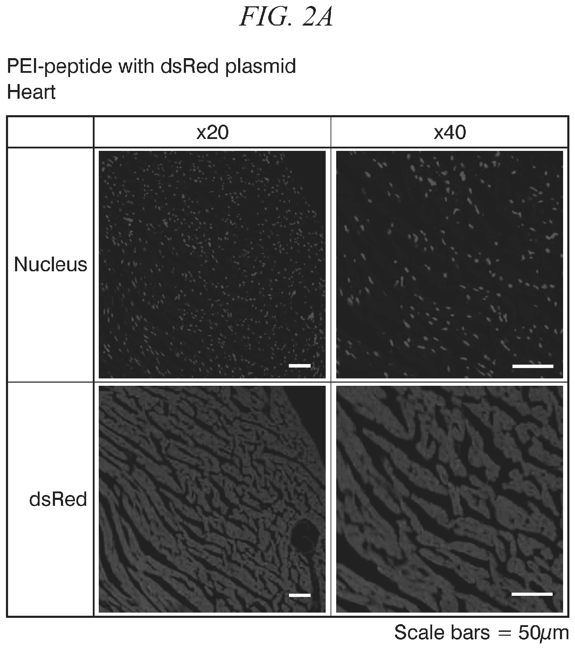

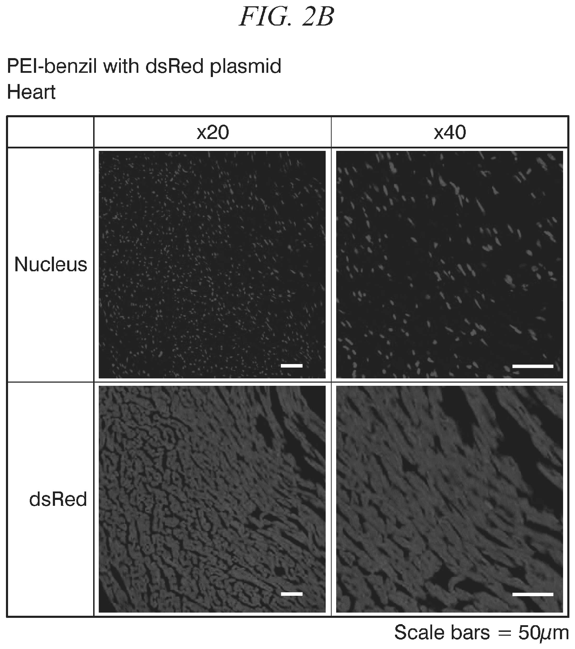

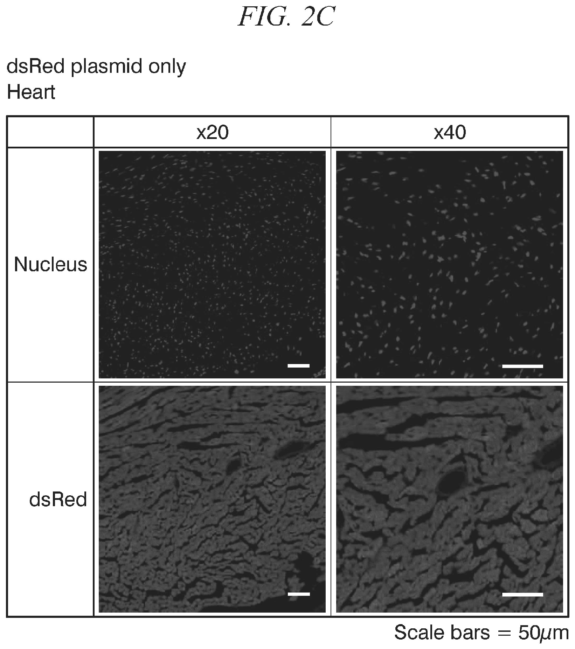

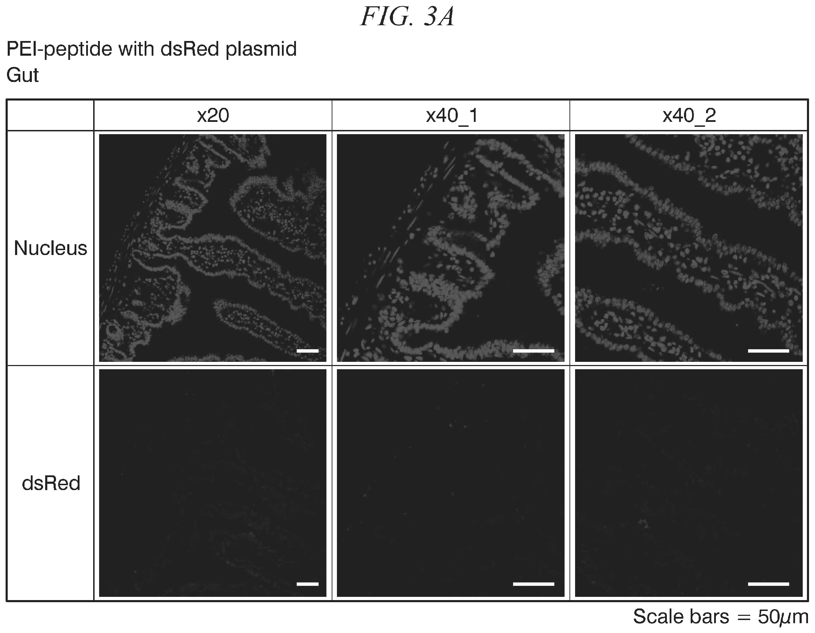

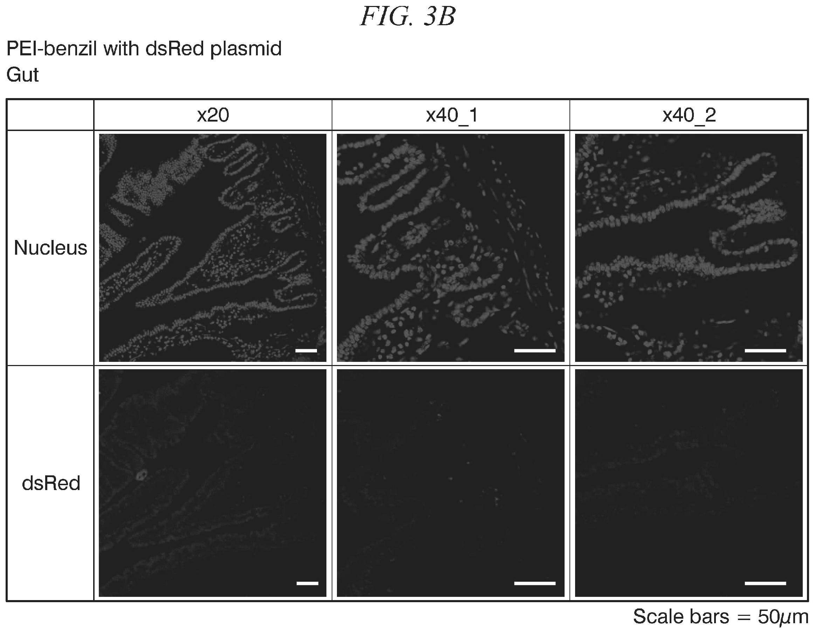

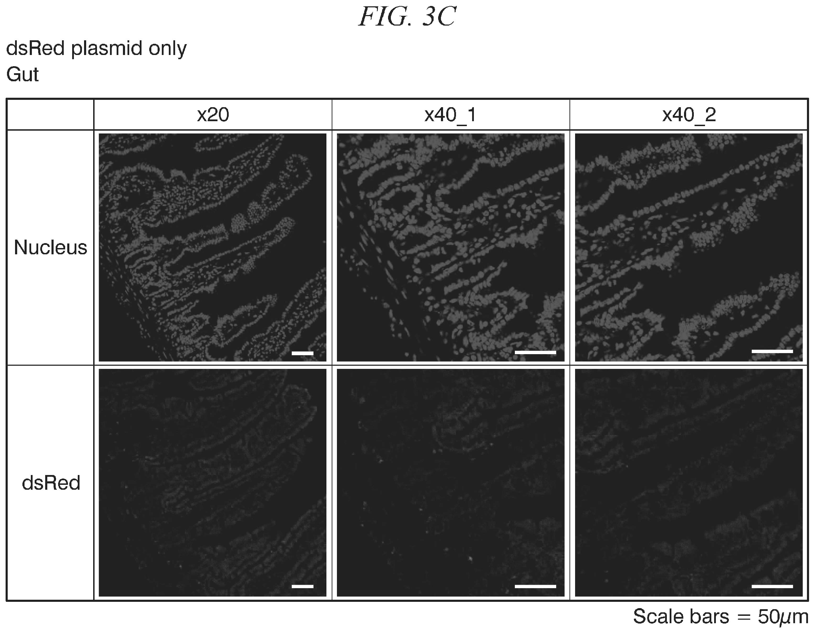

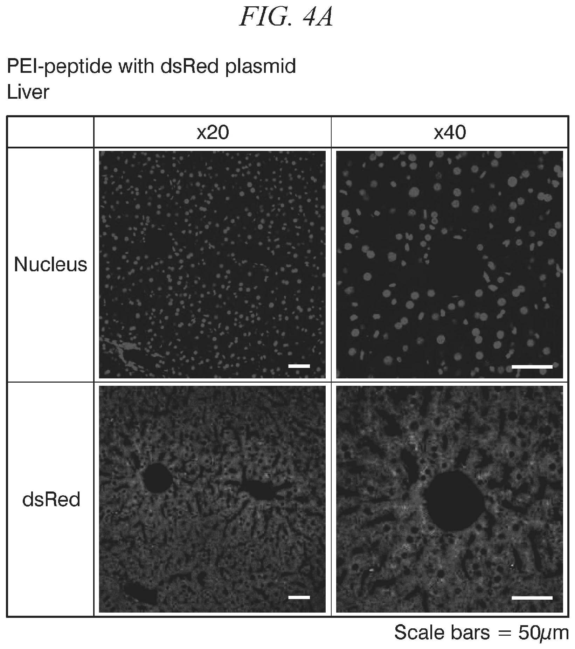

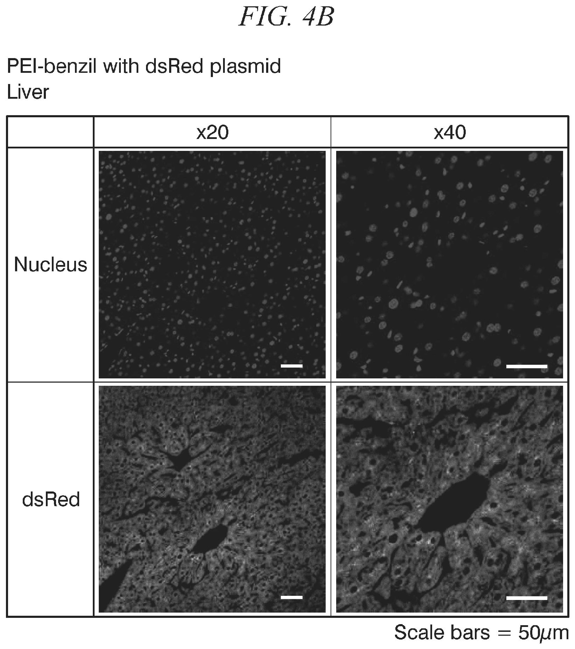

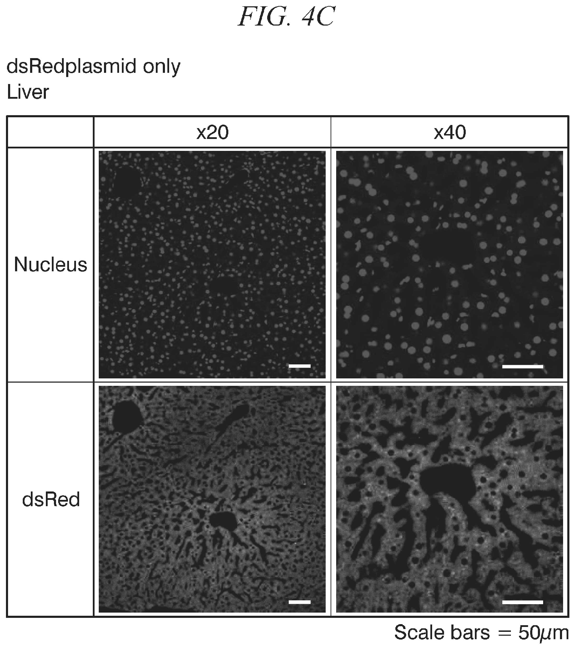

As a result, the expression of dsRed in the brains of mice administered with the nucleic acid-PEI-Benzyl complex was comparable to that of mice administered with dsRed alone, whereas the expression level was significantly higher in mice administered with the nucleic acid-PEI-P_{CN} complex (FIG. 1A-C). Meanwhile, in the heart (FIG. 2A-C), small intestine (FIG. 3A-C), and liver (FIG. 4A-C), the expression levels of dsRed in mice administered with the nucleic acid-PEI-P_{CN} complex were comparable to those of mice administered with the nucleic acid-PEI-Benzyl complex or dsRed alone. These results confirmed that modifying the target nucleic acid with the cerebrum-targeting peptide P_{CN} promotes specific uptake into cerebral tissue.

### INDUSTRIAL APPLICABILITY

The cerebrum-targeting peptide and the transport carrier of the present embodiment are very useful for molecular imaging of cerebral tissue, observation of the condition of cerebral diseases, disease treatment with delivered substances, etc. Furthermore, by further clarifying the state of cerebral tissue using the present invention, it will lead to further progress in methods for diagnosing, preventing, and treating diseases, as well as in personalized medicine, medicine discovery, and elucidation of the causes of diseases.

## Claims

1. A cerebrum-targeting peptide comprising an amino acid sequence represented by SEQ ID NO: 1 (EVGTARY).

2. A transport carrier for specifically transporting to a cerebrum, wherein the transport carrier is a complex in which a cerebrum-targeting peptide comprising an amino acid sequence represented by SEQ ID NO: 1 (EVGTARY) and a cationic polymer are linked.

3. The transport carrier according to claim 2, wherein the cerebrum is a cerebral nerve cell.

4. A pharmaceutical composition, comprising the cerebrum-targeting peptide according to claim 1, the transport carrier according to claim 2, or the transport carrier according to claim 3.

5. The pharmaceutical composition according to claim 4, further comprising a therapeutic agent or a detection agent.

6. A method for screening a peptide that binds to a target cell in a non-human organism, comprising:
contacting a peptide library consisting of 7-amino acid peptides with a target cell to recover a group of peptides that bind to the target cell to obtain a peptide population that binds to the target cell; and
selecting, from the peptide population, a peptide consisting of an amino acid sequence that is identical to a portion of a protein expressed in the non-human organism and that is also identical to a portion of a protein constituting a virus, as a peptide that binds to the target cell.

7. The method for screening a peptide that binds to a target cell according to claim 6, wherein
a group of peptides that specifically bind to the target cells are recovered from the peptide population, and a peptide consisting of an amino acid sequence that is identical to a portion of a protein expressed in the non-human organism and that is also identical to a portion of a ligand recognized by a virus during infection is selected from the group of peptides as the peptide that binds to the target cell.
